(19) Europäisches Patentamt
European Patent Office
Office européen des brevets

(11) EP 0 675 713 B1

(12) **FASCICULE DE BREVET EUROPEEN**

(45) Date de publication et mention de la délivrance du brevet:
**22.03.2000 Bulletin 2000/12**

(21) Numéro de dépôt: **94903924.2**

(22) Date de dépôt: **28.12.1993**

(51) Int. Cl.⁷: **A61K 31/35**, A61K 31/765, A61K 35/78

(86) Numéro de dépôt international:
**PCT/FR93/01312**

(87) Numéro de publication internationale:
**WO 94/14432 (07.07.1994 Gazette 1994/15)**

# (54) UTILISATION DE PRODELPHINIDINES POUR LE TRAITEMENT DE L'ARTHROSE

VERWENDUNG VON PRODELPHIDINEN ZUR BEHANDLUNG VON ARTHROSE

USE OF PRODELPHINIDINES FOR TREATING ARTHROSIS

(84) Etats contractants désignés:
**AT BE CH DE DK ES FR GB GR IE IT LI LU MC NL PT SE**

(30) Priorité: **28.12.1992 FR 9215801**

(43) Date de publication de la demande:
**11.10.1995 Bulletin 1995/41**

(73) Titulaire: **LABORATOIRES DOLISOS**
**31505 Toulouse Cedex 5 (FR)**

(72) Inventeurs:
- **FRANCHIMONT, Paul**
  **B-4577 Modave (BE)**
- **BASSLEER, Corine**
  **39, quai de l'Ourthe**
  **B-4020 Liège (BE)**
- **ANGENOT, Luc**
  **B-4400 Flemalle (BE)**
- **TITS, Monique**
  **B-4041 Vottem (BE)**

(74) Mandataire:
**Martin, Jean-Jacques et al**
**Cabinet REGIMBEAU**
**26, Avenue Kléber**
**75116 Paris (FR)**

(56) Documents cités:
**WO-A-92/14457** **US-A- 4 698 360**

- **PLANTA MED. vol. 57, no. SUPL , 1991 page A131 M. TITS ET AL 'Anti.inflammatory prodelphinidins from black currant (Ribes nigrum) leaves.'**
- **BIOCHEM. PHARMACOL. vol. 33, no. 24 , 1984 pages 3933 - 3939 J.M. TIXIER ET AL 'Evidence by in vivo and in vitro studies that binding of pycnogenols to elastin affects its rate of degradation by elastases.'**
- **TEUSCHER E. 'Pharmazeutische Biologie , Seiten 250 - 251', 1983, FRIEDR. VIEWEG & SOHN, BRAUNSCHWEIG/WIESBADEN**

Remarques:
Le dossier contient des informations techniques présentées postérieurement au dépôt de la demande et ne figurant pas dans le présent fascicule.

EP 0 675 713 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet l'utilisation de prodelphinidines, et plus particulièrement celles obtenues à partir de Ribes species, notamment Ribes nigrum, pour l'obtention de médicaments destinés au traitement de l'arthrose.

Dans les conditions normales, le cartilage articulaire est soumis à un remodelage lent assuré par un mécanisme de chondrorésorption parfaitement compensé par un processus de chondroréparation (ou chondroformation) qui maintient le cartilage articulaire en équilibre dynamique (Franchimont et al., 1991).

La chondrorésorption est assurée par le chondrocyte lui-même qui produit des prostaglandines dont la PGE2, des enzymes protéolytiques (collagénase, stromelysine, serine protéases...) et des radicaux libres oxygène qui s'attaquent à la matrice cartilagineuse, la désorganise et la détruit.

Parallèlement à cette chondrorésorption, a lieu une chondroformation homéostatique qui répare la matière altérée et/ou résorbée; il y a production des macromolécules qui forment la structure de la substance fondamentale: les collagènes de type II et de type IX, les protéoglycanes, les protéines de jonction, etc... On peut assister à la prolifération clonale de chondrocytes lorsque la chondroformation doit être importante. Cette phase de chondroformation est un processus dépendant de plusieurs facteurs dont la concentration en macromolécules des zones de voisinage du chondrocyte et de l'équilibre hormonal, en particulier de la sécrétion d'hormone de croissance, d'IGF I et II, de calcitonine, d'androgènes...

Différents facteurs pathogéniques peuvent survenir qui accélèrent la dégradation du cartilage par accélération du processus de chondrorésorption rompant l'équilibre entre la chondrorésorption et la chondroréparation en faveur du premier. Il s'agit, entre autre, des surcharges fonctionnelles et pondérales articulaires, des dépôts de microcristaux et des troubles de nutrition des structures articulaires. Ces facteurs pathogéniques vont agir en stimulant la production de cytokines (IL1 $\beta$, TNF $\alpha$, IL6...) par les chondrocytes eux-mêmes, par d'autres cellules des structures de voisinage comme les synoviocytes A de la synoviale articulaire. Le déséquilibre sera d'autant plus net qu'il existera des perturbations hormonales (comme c'est le cas après 50 ans, âge du développement de l'arthrose) rendant la chondroréparation moins efficace.

Ce déséquilibre biochimique conduit à une fragilisation du cartilage articulaire qui se fissure d'abord en superficie puis en profondeur mettant à nu l'os sous chondral. L'arthrose est l'expression clinique de cette perte de fonction cartilagineuse, elle-même conséquente du déséquilibre biochimique entre la chondrorésorption et la chondroréparation.

La thérapeutique médicamenteuse de l'arthrose est pauvre: les anti-inflammatoires non stéroïdiens luttent contre la douleur et améliorent le mouvement; ils n'arrêtent pas l'évolution de la maladie, en particulier la destruction du cartilage, et ne permettent pas la réparation du cartilage.

Notamment, l'utilisation de pycnogénols provenant d'extraits de végétaux tels que Ribes nigrum, pour la préparation de médicaments à activité anti-inflammatoire, fait l'objet de la demande internationale WO 92/14457 du 14 février 1992.

Par ailleurs, l'utilisation de proanthocyanidines pour la prévention ou le traitement de pathologies dues aux effets biologiques des radicaux libres, notamment des inflammations, fait l'objet du brevet US n° 4, 698, 360 déposé le 9 avril 1985.

La présente invention a précisément pour but de fournir des médicaments qui, par leurs propriétés de réduction de la chondrorésorption et de stimulation de la chondroréparation, permettent de traiter efficacement l'arthrose ou tout autre pathologie directement ou indirectement liée à une destruction du cartilage et à l'absence de réparation de ce dernier par les mécanismes biologiques de l'organisme.

La présente invention a pour objet l'utilisation de prodelphinidines de formule (I)

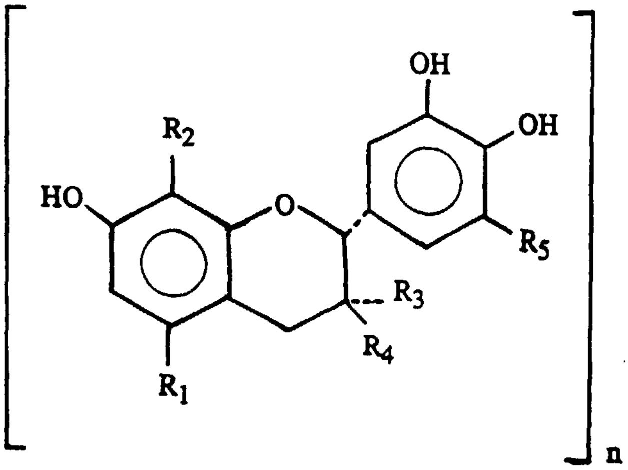

(I)

dans laquelle:

- $R_1$ et $R_2$ représentent indépendamment H ou OH,
- $R_3$ et $R_4$ sont différents et représentent H ou OH,
- $R_5$ représente H (catéchine/épicatéchine) ou OH (gallocatéchine/épigallocatéchine), et,
- n est un nombre entier de 2 à 40,
- sous réserve que l'une au moins des 2 à 40 unités décrites ci-dessus, correspond à une gallocatéchine ($R_3$ = H, $R_4$ = OH), ou à une épigallocatéchine ($R_3$ = OH, $R_4$ = H), pour laquelle $R_5$ représente OH, pour l'obtention de médicaments destinés au traitement des pathologies articulaires liées à une destruction du cartilage, et plus particulièrement de l'arthrose.

L'invention a pins particulièrement pour objet l'utilisation susmentionnée des prodelphinidines choisies parmi celles répondant à la formule (II) suivante:

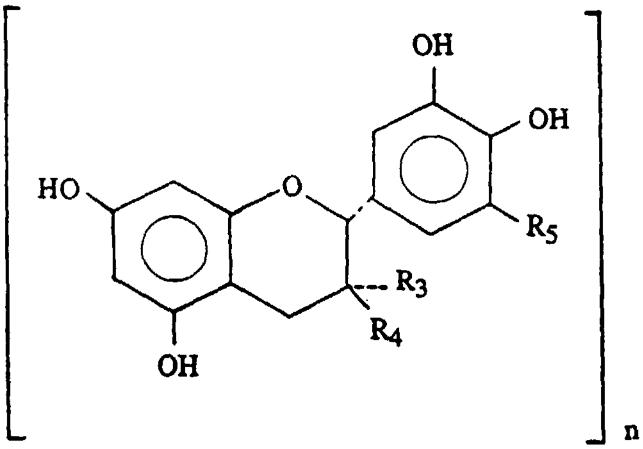

(II)

dans laquelle $R_3$, $R_4$ et $R_5$ sont tels que définis ci-dessus dans le cadre de la formule (I).

Par leurs propriétés de stimulation de la production de protéoglycanes et de collagènes (notamment de type II) dans le cartilage, les prodelphinidines selon l'invention sont susceptibles de restaurer le processus de chondroformation.

Ces prodelphinidines présentent en outre l'avantage de réduire la chondrorésorption, notamment par leurs effets de réduction de la production des prostaglandines (notamment du type E2) et de réduction de la production de radicaux libres dans l'organisme.

Les médicaments selon l'invention sont plus particulièrement destinés à l'administration par voie orale, parentérale ou topique.

De préférence, les prodelphinidines contenues dans ces médicaments sont celles issues de plantes, et plus particulièrement de Ribes species, notamment de Ribes nigrum.

Les plantes à partir desquelles lesdites prodelphinidines sont isolées, sont obtenues à partir des méthodes classiques en champs, ou bien par des techniques de production in vitro.

Selon un mode de réalisation de l'invention, les médicaments susmentionnés comprennent des extraits de Ribes species, et plus particulièrement de Ribes nigrum, ces extraits comprenant eux-mêmes des prodelphinidines.

Ces extraits de Ribes, notamment de Ribes nigrum peuvent avantageusement être obtenus selon les procédés suivants.

Dans un premier procédé avantageux pour préparer un extrait de Ribes, on pulvérise un lot de feuilles que l'on épuise par percolation lente au moyen d'éthanol à 70°. Après obtention du dernier percolat, on exprime la poudre et on rassemble toutes les solutions extractives. L'éthanol est éliminé par distillation à l'évaporateur rotatif, sous pression réduite, à une température inférieure à 50°C. La solution aqueuse restante est filtrée afin d'éliminer une partie importante de chlorophylle. On pratique ensuite un fractionnement en utilisant successivement des solvants de polarité croissante, tels que:

a) l'éther diéthylique afin d'éliminer la chlorophylle restante et diverses substances liposolubles;
b) l'acétate d'éthyle;
c) le n-butanol.

Dans un autre procédé avantageux d'extraction, on épuise un lot de poudre de feuilles sèches de Ribes par percolation lente, au moyen d'un mélange acétone-eau. Après obtention du dernier percolat, on exprime la poudre à la presse et on rassemble tous les liquides. L'extrait total peut être agité vigoureusement avec du NaCl, ajouté jusqu'à l'obtention de deux phases: une phase supérieure acétonique et une phase inférieure aqueuse. La phase acétonique est évaporée sous pression réduite à 30°C et le résidu aqueux est additionné d'un égal volume d'eau, filtré puis ré-extrait: soit par de l'acétate d'éthyle, soit par du butanol. Les extraits sont ensuite évaporés à sec.

On obtient ainsi des extraits contenant au moins 2% en poids de prodelphinidines de formule (I) ou (II).

Selon un autre mode de réalisation particulièrement avantageux de l'invention, les prodelphinidines contenues dans les médicaments susmentionnés, sont isolées et purifiées à partir d'extraits de plantes susceptibles de contenir lesdites prodelphinidines, et plus particulièrement à partir de plantes appartenant à Ribes species, notamment Ribes nigrum.

L'isolement et la purification des prodelphinidines à partir des plantes susmentionnées, et plus particulièrement à partir de Ribes nigrum, est avantageusement effectuée par différentes techniques de chromatographie sur gel, ou encore des techniques d'ultra-filtration connues de l'homme du métier, à partir des extraits obtenus selon les procédés décrits ci-dessus.

Parmi les techniques chromatographiques, on citera notamment l'utilisation de la colonne Fractogel TSK® (MERCK, USA) en éluant à l'aide d'eau, puis d'un mélange eau/méthanol à pourcentage croissant en méthanol; la colonne Séphadex LH 20® (PHARMACIA, SUEDE) en éluant à l'aide d'alcool puis d'alcool additionné d'eau; ou encore sur une colonne Lobar Lichroprep RP8® (MERCK, USA), l'élution étant réalisée à l'aide d'un mélange eau/acétone.

Ces différentes techniques peuvent être combinées entre elles pour obtenir un plus haut degré de purification.

Des médicaments particulièrement préférés dans le cadre de la présente invention sont caractérisés en ce qu'ils contiennent des prodelphinidines choisies parmi l'une au moins des molécules (Ia), (Ib), et (Ic) suivantes:

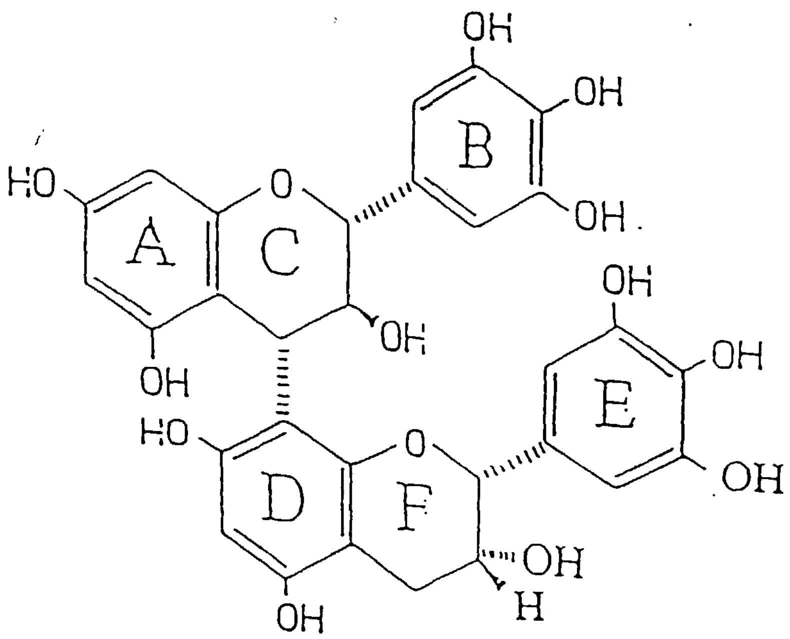

(Ia)

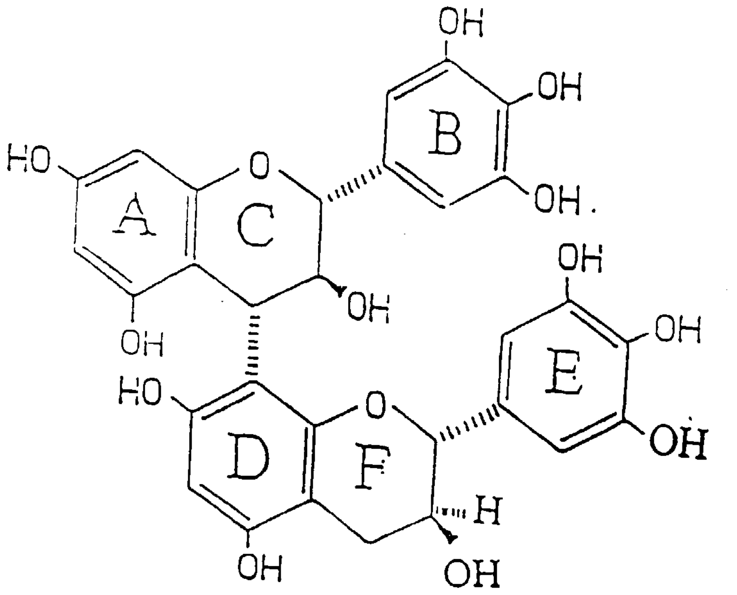

(Ib)

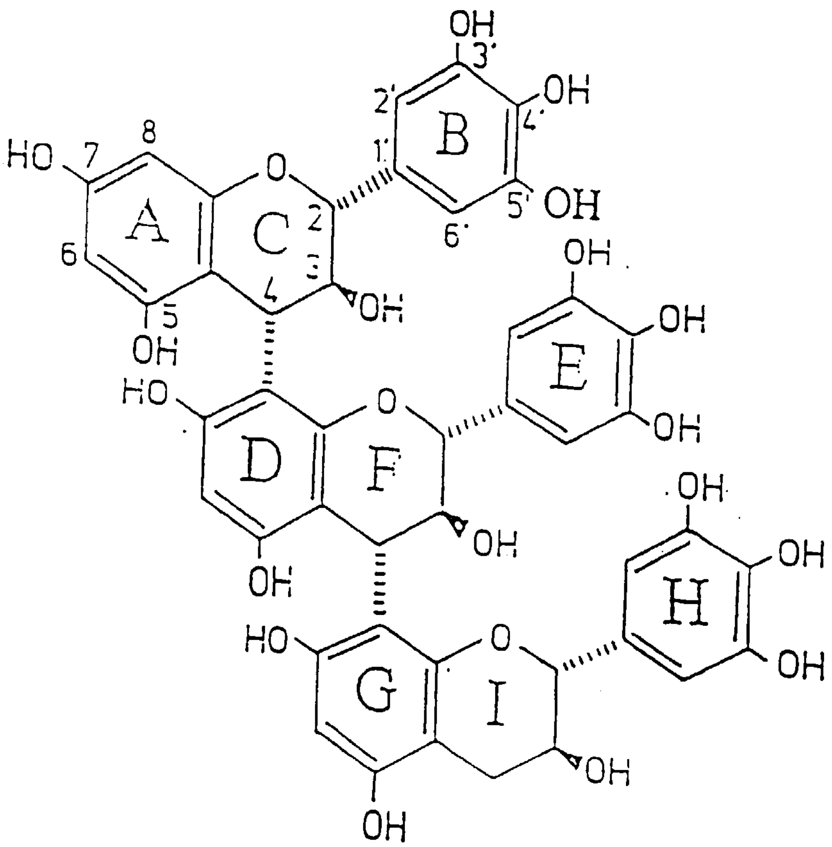

(Ic)

L'invention sera davantage illustrée dans la description détaillée qui suit de l'effet de compositions à base de prodelphinidines selon l'invention, sur la chondroréparation et la chondrorésorption.

Le modèle utilisé pour tester les proanthocyanidols à savoir la culture des chondrocytes humains en trois

dimensions permet de quantifier la chondroréparation par la production de substances matricielles: collagène de type II, protéoglycanes, et la chondrorésorption par la réduction des taux produits de la prostaglandine E2 et des radicaux libres de type OH° et O°$_2$.

I) MATERIELS ET METHODES (Franchimont et al., 1989)

**1.** Culture tridimensionnelle de chondrocytes humains

La technique utilisée a été décrite en détail (Bassleer et al., 1986).

Brièvement, des chondrocytes articulaires humains sont cultivés dans le DMEM (Dulbecco's Modification of Eagle's Medium - ICN - Gand, Belgique) additionné soit d'Ultroser G 1% (GIBCO, Gand, Belgique) soit de 10% de sérum de veau foetal (ICN - Gand - Belgique), et de 50 µg d'acide ascorbique/ml. Le cartilage provient de la zone apparemment normale de têtes fémorales humaines atteintes d'arthrose, immédiatement après l'opération réalisée pour la mise en place d'une prothèse totale de hanche; il peut également provenir de tête fémorale saine, mais ayant subi un traumatisme et devant être remplacée. Ce cartilage est ensuite digéré par la collagénase clostridiale 1 mg/ml (Boehringer-Mannheim, Allemagne) dans un tampon, (NaCl 120 mM, $CaCl_2$ 1mM, KCL 5 mM, $KH_2PO_4$ 1 mM, $NaHCO_3$ 25 mM, glucose 2 mM et Hepes 30mM, pH 7.4) durant 24 heures. Après 6 lavages successifs et centrifugations, les chondrocytes, isolés de leur matrice, sont mis en culture dans des flacons ($10^6$ cellules/2 ml de milieu de culture) placés sur un gyrotor (100 tpm). Les cultures sont maintenues à 37 °C dans une atmosphère air 95%-CO2 5% (Bassleer et al., 1986). Cultivés dans ces conditions, après 4 à 5 jours, les chondrocytes forment un agrégat tridimensionnel dans chaque flacon. Ces agrégats, d'abord d'aspect floconneux, tendent ensuite à se condenser au cours de la culture. Après deux semaines, leur diamètre est, en général, de 1 à 2 mm.

Le poids frais des agrégats augmente avec la durée de culture, surtout durant les premiers jours.

**2.** Dosage radioimmunologique des protéoglycanes (PG) cartilagineux

Les PG sont mesurés par un dosage radioimmunologique (RIA) spécifique et sensible des PG cartilagineux humains (Gysen et al., 1984). Les PG ont été extraits à partir de cartilage humain, selon les méthodes décrites par Roughley et al. (1981) et par Bayliss et Venn (1980). L'antisérum contre les PG cartilagineux humains a été obtenu chez le lapin selon la technique de Vaitukaitis et al. (1971). Le dosage radio-immunologique est réalisé dans un volume de 0,4 ml consistant en 0,1 ml de la solution traceur (15000 à 20000 cpm $^{125}$I-PG, marqués par la méthode à la chloramine T (Greenwood, 1973), 0,1 ml d'antisérum anti-PG dilué à 1/5000 dans un tampon phosphate salin (PBS - $PO_4$ 50 mM, NaCl 150 mM), contenant de la BSA (albumine de sérum bovin-5g/l et de l'azide sodium -6.7 mM) (tampon d'incubation) et 0,2 ml de plusieurs dilutions de milieu de culture ou d'extrait d'agrégat ou encore de l'antigène (0,5 - 500 ng/tube).

Après quatre jours à 4°C, les PG* (PG radioactifs) libres sont séparés des complexes PG*-anticorps par double précipitation (Franchimont et al., 1983).

La sensibilité du dosage est de 0,6 ng/tube. L'exactitude de la mesure est de l'ordre de 4%. Les coefficients de variation intra- et inter dosages sont de moins de 10 % et de 20 % respectivement.

Les anticorps sont dirigés contre les déterminants antigéniques de la protéine centrale du PG. Il existe une réaction croisée complète avec les PG humains de cartilage de côte, de disque vertébral et de trachée, de même avec les PG extraits de veine ou d'artère. Il n'y a pas de réaction croisée avec les glycosaminoglycanes ou avec les PG extraits de tissus foetaux ou les petits PG de l'os. De plus, le dosage est spécifique de l'espèce, puisque les PG extraits de cartilage de chien, de rat, de poulet ou de veau embryonnaires ne réagissent pas dans le dosage. Le collagène de type II, la fibronectine, le sulfate de chondroitine et l'acide hyaluronique n'interfèrent pas dans le dosage.

Les PG sont mesurés directement dans les milieux de culture. Les agrégats de chondrocytes, quant à eux, sont lavés 3 fois dans un tampon phosphate salin (PBS- $PO_4$ 50 mM, NaCl 150 mM, pH 7,4) contenant des inhibiteurs de protéases (Oegema et al., 1975: acide-6-aminohexanoique 100 mM, EDTA 10 mM, chlorhydrate de benzamidine 50 mM, inhibiteur de trypsine $5.10^{-8}$ M, azide de sodium 6,7 mM et de la superoxyde dismutase 200 U/ml). Dans ce même tampon, les agrégats sont homogénéisés par dissociation aux ultrasons (10 secondes, à 4° C, puissance: 200 watts/cm2). Cette extraction permet d'obtenir 70 à 80 % des PG extraits par HCL-guanidine.

**3.** Dosage radioimmunologique du collagène de type II (coll.II)

La méthode de dosage radioimmunologique du coll.II a été décrite en détail (Henrotin et al., 1990). Le coll.II est extrait du cartilage articulaire humain selon la technique décrite par Herbage et al.(1977). L'antisérum conte le coll.II a été obtenu chez le lapin selon la technique de Vaitukaitis et al. (1971).

Le dosage est réalisé par saturation séquentielle. Dans un premier temps, le dosage radioimmunologique est

réalisé dans un volume de 0,3 ml consistant en 0,2 ml de tampon d'incubation (PBS: $PO_4$ 50 mM, NaCl 300 mM, BSA 5g/l (P/V), azide de sodium 6,7 mM, pH 7,4) contenant le coll.II de référence (0,5 à 500 ng) ou l'échantillon à doser et 0,1 ml d'antisérum anti-coll.II de cobaye dilué à 1/5000 (24 h à 4° C). Dans un deuxième temps, 0,1 ml de traceur $^{125}$ I-coll.II marqué selon la technique à i'iodogène (Salacynski et al., 1979 et dilué de manière à obtenir 20.000 cpm/0,1 ml) est ajouté. Après 24 heures à 4° C, les complexes collagène-anticorps sont séparés du coll.II radioactif libre par le système à double anticorps (Franchimont et al. 1983). La saturation séquentielle permet une sensibilité de 3 ng/tube. L'exactitude de la méthode est de 10%. Les coefficients de variation intra- et interdosages sont de 10 et 20% respectivement.

Le dosage est spécifique du collagène de type II articulaire humain. Il n'y a pas de réaction croisée avec d'autres constituants du cartilage: PG humains, fibronectine, laminine et acide hyaluronique n'interfèrent pas dans le dosage.

Aucune réaction croisée n'est obtenue avec le collagène bovin de type I, III ou IV.

Les milieux de culture sont conservés à - 20° C en présence d'inhibiteurs enzymatiques (Oegema et al., 1975). Ces milieux sont dosés par la méthode radioimmunologique sans traitement préalable. Les agrégats de chondrocytes sont lavés et dissociés aux ultrasons selon la méthode décrite pour le dosage des PG. Après centrifugation (1500 tpm, 10 minutes), le résidu est extrait à l'aide d'acide acétique 500 mM sous agitation durant 24 heures à 4° C. Le surnageant de l'homogénat et l'extrait du résidu sont mélangés et dosés après équilibration à pH 7,4 à l'aide de NaOH 4M.

La récupération du coll.II est de 88 ± 8 % (x ± 1 d.s.).

**4.** Dosage radioimmunologique des prostaglandines E2 (PGE2)

Le dosage radioimmunologique des PGE2 est réalisé selon la méthode décrite par Serteyn et al., 1988. La PGE2 est obtenue chez Sigma (Aldrich-Chemie, Steinheim, Allemagne) et la $^3$ H-PGE2 chez NEN (Du Pont de Nemours, Bruxelles, Belgique). Aux 0,1 ml de l'échantillon à doser ou de l'antigène de référence (0-500 pg) sont ajoutés, successivement, 0,1 ml de tampon d'incubation (Tris-HCL 10 mM, NaCl 150 mM, azide de sodium 0,5% (P/V), gélatine 1% (P/V), pH 7,4), 0,1 ml de $^3$ H-PGE2 (dilué de manière à obtenir 10.000 cpm/0,1 ml) et 0,1 ml d'antisérum (1/4000). Après 48 heures d'incubation à 4°C, l'antigène libre est séparé des antigènes liés aux anticorps par une précipitation au charbon - DEXTRAN T70 (0,5 ml par tube). La solution de charbon - Dextran contient 5g/l de charbon neutre, 500 mg de Dextran 70/l. Après centrifugation (20 minutes, 3000 tpm, 4°C), la radioactivité présente dans le surnageant est mesurée au compteur bêta (Beckman) par scintillation liquide. La sensibilité du dosage est de 20 pg/ml.

L'exactitude de la méthode est de 5%. Les coefficients de variation intra-et interdosages sont de 6 et de 10% respectivement. Il n'y a pas de réaction croisée entre cet antisérum anti PGE2 et d'autres prostanoides (Thromboxane B2, G-keto-PGF1-α, PGF2, PGA2), ni avec des acides gras tels que l'acide arachidonique, l'acide oléique ou encore l'acide linoléique.

**5.** Analyse de la synthèse de DNA par la mesure de l'incorporation de $^3$H-thymidine

Après différentes durées de culture, les chondrocytes sont cultivés dans le milieu nutritif additionné de 2 uCi/ml (5 Ci/mmole) de méthyl $^3$H-thymidine ($^3$H-TDN) (Amersham, Bruxelles, Belgique) durant 24 heures. Les agrégats de chondrocytes sont ensuite lavés pendant 20 minutes à l'aide d'un tampon phosphate salin (PBS: $PO_4$ 50 mM, NaCl 150 mM, pH 7,4) contenant les mêmes inhibiteurs de protéases que ceux utilisés pour le dosage des PG. Ils sont ensuite incubés pendant 20 minutes dans du PBS contenant de la thymidine froide (100 µg/ml), puis lavés à nouveau pendant 20 minutes, deux fois.

L'agrégat est alors dissocié par les ultrasons (10 sec., 4°C, 200 watts/cm2).

La radioactivité incorporée dans l'agrégat est ensuite mesurée à l'aide d'un compteur bêta à scintillation.

**6.** Dosage de l'ADN

Le dosage du contenu en ADN des agrégats est réalisé sur les agrégats dissociés par ultrasons, selon la méthode fluorimétrique décrite par Labarca et Paigen (1980).

Sur un échantillon d'agrégats homogénéisés aux ultrasons, le réactif fluorochrome de bisbenzimide (FB) (Hoechst n° 33258 - Calbiochem-Behring Cor - La Jolla - Californie) est ajouté (4g/tube). La fluorescence des complexes ADN-FB est mesurée aussitôt au fluorimètre (longueur d'onde d'excitation: 256 nm, longueur d'onde d'émission: 458 nm). De l'ADN bovin (0-5 µg) est utilisé comme référence. La fluorescence des complexes est directement proportionnelle à la concentration en ADN.

**7.** Calculs et analyse statistique

Les résultats sont exprimés en quantités de PG, coll.II ou PGE2 mesurées dans le milieu de culture (MC) par µg d'ADN. Les mesures cumulatives sont obtenues en ajoutant les quantités dosées dans les MC à chaque renouvellement du MC, pour des périodes successives: 0 à 4, 4 à 8, 8 à 12 jours de culture. La production totale de PG est calculée en ajoutant les quantités cumulées mesurées dans les MC à celles mesurées dans les agrégats correspondants. La moyenne et la déviation standard (m +/- d.s) sont calculées. La comparaison des valeurs moyennes est réalisée par le test statistique U de Mann- Whitney.

**8.** Production de radicaux hydroxyle OH°

Principe de la méthode:

Les produits sont dissous dans des flacons de 10 ml à la concentration requise dans du tampon phosphate: 6,6 $10^{-2}$ M, p H 7.4 en présence d'acide α keto methiolbutyrique ($10^{-3}$ M: KMB). Après la fermeture hermétique des flacons, les échantillons sont irradiés par rayons γ fournis par une source de $^{137}$ Cs.

Les radicaux OH° produits par irradiation γ réagissent avec le KMB pour produire de l'éthylène détecté par un chromatographe en phase gazeuse. La hauteur du pic d'éthylène est proportionnelle à la quantité de radicaux libres formés dans le milieu (Weis et al., 1978).

**9.** Activité antilipoperoxydante

Principe de la méthode:

Semblable à la méthode antiradicalaire précédente mais où le KMB est remplacé par l'acide linoléique (C18: 2), l'irradiation γ libère du pentane qui est dosé. La hauteur du pic de pentane est proportionnelle à la quantité de radicaux libres formés dans le milieu.

**10.** Production d'anion superoxyde ($O°_2$) par des cellules polymorphonucléaires (PMNs).

Principe de la méthode:

Celle-ci est mesurée avec un spectrophotomètre à double faisceau qui permet de suivre la réduction du ferricytochrome C par $O°_2$. Le milieu d'incubation (2,5 ml) est constitué de tampon PBS contenant du glucose (7,5 mM), du ferricytochrome C (0,056 mM), du $CaCl_2$ (2,3 mM), du $MgCl_2$ (1,1 mM), $7{,}5.10^6$ de cellules et la substance à tester à une concentration de 0,032 mM. La réaction est enclenchée en ajoutant du phorbol myristate acétate à une concentration finale de 0,012 mM et la réduction du cytochrome C est suivie à 550 nm pendant 10 minutes. La cuvette de référence contient le même matériel que celui décrit ci-dessus avec en plus 48µg de superoxyde dismutase (SOD)/ml de milieu.

II <u>RESULTATS CONCERNANT LES PRODUITS DE FORMULE (Ia)</u> <u>(Ib) et (Ic)</u>:

Ia GC-(4 α -> 8) EGC

Ib GC-(4 α -> 8) GC

Ic GC-(4 α -> 8) GC-(4 a -> 8)-GC

GC et EGC représentant respectivement la gallocatéchine et l'épigallocatéchine de formules suivantes

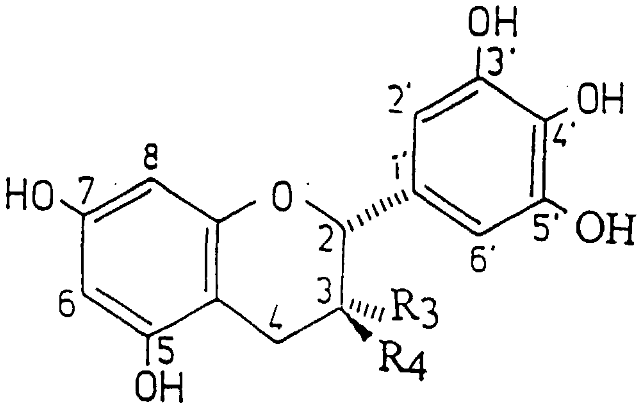

| | R3 | R4 |
|---|---|---|
| GC | H | OH |
| EGC | OH | H |

II-1 Incorporation de $^{3}H$ thymidine dans les chondrocytes, indice de prolifération cellulaire

Aucune des substances Ia, Ib et Ic ne modifie l'incorporation de $^{3}H$ thymidine dans les chondrocytes aux trois doses testées 1, 10 et 100 µg/ml par rapport aux valeurs témoins.

II-2 Dosage des protéoglycanes du collagène de type II et des protaglandines E2

Les expérimentations ont été réalisées trois fois à plusieurs mois d'intervalle dans deux conditions expérimentales différentes:

(1) en l'absence de serum de veau foetal mais en milieu synthétique: 1% ULTROSER G
(2) en présence de 5% de serum de veau foetal.

A. EN PRESENCE D'ULTROSER

1. Proteoglycanes (PG)

Les taux de PG libérés dans les milieux de culture et rapportés au µg d'ADN pendant chaque période d'incubation de 4 jours (µg/µg ADN/96 h) sont repris dans les tableaux 1,2,3. Exprimé en taux cumulés absolus et pourcentuels, il apparaît que l'effet des trois substances est assez semblable entre elles (tableau 4).
Il faut cependant remarquer que seule, la substance Ic présente un effet significatif à la dose de 1 µg/ml (tableau 3).
Les concentrations de PG dans les agrégats sont significativement stimulés et de façon dose dépendante par les trois concentrations étudiées des substances Ia et Ic; pour la substance Ib, aucun effet significatif n'est noté pour la concentration de 1 µg/ml (tableaux 5,6,7).

2. Collagène de typeII

Les taux de collagène de type II libéré dans le milieu de culture par période d'incubation de 4 jours et exprimé par µg d'ADN (ng/µg ADN/96 h) sont illustrés dans les tableaux 8 (substance Ia) 9 (substance Ib) et 10 (substance Ic). Seule, la substance Ic exerce un effet significatif à la plus faible dose expérimentée: 1 µg/ml. La libération cumulée en 12 jours exprimée en valeur absolue et en valeur pourcentuelle est reprise dans le tableau 11. Elles sont pratiquement

identiques pour les trois substances Ia, Ib et Ic.

Les valeurs de collagène II dans les agrégats de 12 jours exprimées en ng par µg d'ADN dans les agrégats de 12 jours sont repris dans le tableau 12. La substance Ic est la seule à être stimulante à la dose de 1µg/ml.

3. Prostaglandine E2 (PGE2)

Les prostaglandines ont été dosées dans les milieux de culture correspondant à l'incubation du 4e au 8e jour, période où l'agrégat est formé.

Les trois substances réduisent significativement le taux des PGE2 (tableau13).

B. EN PRESENCE DE S.V.F.

Deux expériences (cultures 397 et 403) ont été réalisées pour confirmer les résultats. Elles ont été allégées, d'une part, en réalisant les cultures pendant 8 jours au lieu de 12 jours et, d'autre part, deux doses seulement des produits Ia, Ib et Ic ont été étudiées: 10 et 100 µg/ml.

1. Protéoglycanes (PG)

Les PG libérés en milieu de culture et exprimés en µg/µg d'ADN/96 H sont significativement accrus en présence de 10 et 100 µg/ml. Un effet dose-réponse est observé pour les trois substances (tableaux 14 et 15).

Les PG dans les agrégats sont également significativement accrus lors de l'incubation des substances Ia, Ib et Ic aux concentrations de 10 et 100 µg/ml.

Un effet dose-dépendant est observé (tableaux 16 et 17). Ces résultats sont observés pour les deux expériences réalisées : cultures 397 et 403.

2. Collagène de type II

Le collagène de type II s'accroît dans les milieux de culture lorsque les chondrocytes sont incubés avec les substances Ia, Ib et Ic aux concentrations de 10 et 100 µg/ml. La réponse est dose-dépendante (tableaux 18 et 19). Dans les agrégats, un accident a eu lieu qui n'a permis de doser le collagène II qu'au cours de l'expérience 397. On observe un accroissement de collagène II pour les 3 substances Ia, Ib et Ic aux concentrations de 10 et 100 µg/ml. L'effet est dose-dépendant (tableau 20).

3. Prostaglandines E2 (PGE2) (tableau 13)

Un effet inhibiteur significatif est observé pour les trois substances à 10 et 100 µg/ml (sauf pendant la première période d'incubation jour 0-4 pour 10 µg/ml de la substance Ia) durant les deux périodes d'incubation de 0 au 4e jour et du 4e au 8e jour (tableau 21).

II-3. Production de radicaux hydroxyle OH°

Résultats:

L'ajout de substance Ia, Ib et Ic inhibe significativement la production de radicaux libres à partir du KMB (tableau 22) ou à partir de l'acide linoléique (tableau 23). Aux doses de $10^{-5}$ M les substances testées ont encore une action sur le modèle au KMB. Par contre, elles sont sans effet sur le modèle utilisant l'acide linoléique comme substrat.

II-4. Production d'anion superoxyde ($O°_2$) par des cellules polymorphonucléaires (PMNs).

Résultats:

En présence de substances Ia et Ib, la réduction du ferricytochrome C est fortement diminuée suggérant ainsi que le produit agit soit en piégeant l'anion superoxyde, soit en inhibitant le système enzymatique des PMNs responsable de la production de cette espèce oxygénée activée. Cependant, des expériences témoins indiquent que le produit La ainsi que Ib, Ic et l'esculoside sont par eux-mêmes de puissants réducteurs du ferricytochrome C. Cet effet n'étant pas quantifiable, il ne peut être tiré par conséquent aucune conclusion sur un éventuel effet inhibiteur de ces produits sur la production d'anion superoxyde par les PMNs activés.

III - Conclusion de l'étude des proanthocyanidols sur le modèle de chondrocytes humains cultivés en trois dimensions

Les substances Ia, Ib et Ic agissent sur le modèle étudié en:

1. augmentant significativement la production de protéoglycanes, dosés par méthode radio-immunologique, dans le milieu de culture et dans l'agrégat néoformé;
2. augmentant significativement la production de collagène de type II, dosé par méthode radio-immunologique, dans le milieu de culture et dans l'agrégat néoformé;
3. réduisant significativement la production des prostaglandines E2 dans les milieux de culture pendant les deux première périodes de culture du 1er au 4ème jour, et du 4ème au 8ème jour;
4. réduisant significativement les radicaux libres OH° produits en présence soit de MKB, soit d'acide linoléique.

Les résultats ont été reproduits au cours de trois expériences consécutives dans deux conditions de milieu de culture: en présence d'Ultroser 1% et en présence de sérum de veau foetal.

Ces substances ont un profil de médicament chondroprotecteur.

Les tableaux indiqués ci-dessus sont les suivants:

TABLEAU 1

| PG LIBERES DANS LE MILIEU DE CULTURE (µg/µg ADN/96 h) | | | | |
|---|---|---|---|---|
| TEMPS DE CULTURE (JOURS) | SUBSTANCE Ia (µg/ml) | | | |
| | 0 | 1 | 10 | 100 |
| 0 - 4 | 13,6 ± 1,6 | 14,2 ± 1,6 | 22,4 ± 0,2* | 28,6 ± 2,85* |
| 4 - 8 | 1,96 ± 0,24 | 1,74 ± 0,18 | 3,26 ± 0,12* | 3,94 ± 0,1* |
| 8 - 12 | 0,42 ± 0,03 | 0,44 ± 0,04 | 0,74 ± 0,18* | 0,78 ± 0,02* |

* : p < 0,025 comparé aux témoins

TABLEAU 2

| PG LIBERES DANS LE MILIEU DE CULTURE (µg/µg ADN/96 h) | | | | |
|---|---|---|---|---|
| TEMPS DE CULTURE (JOURS) | SUBSTANCE Ib (µg/ml) | | | |
| | 0 | 1 | 10 | 100 |
| 0 - 4 | 13,7 ± 1,66 | 13,0 ± 1,22 | 20,4 ± 3,0* | 28,2 ± 2,92* |
| 4 - 8 | 3,14± 0,14 | 3,45 ± 0,56 | 4,78 ± 0,56* | 5,65 ± 0,24* |
| 8 - 12 | 0,71 ± 0,09 | 0,72 ± 0,08 | 1,00 ± 0,12* | 1,32 ± 0,01* |
| : p < 0,025 comparé aux témoins<br>(U - test de Mann-Whitney) | | | | |

TABLEAU 3

| PG LIBERES DANS LE MILIEU DE CULTURE (µg/µg ADN/96 h) | | | | |
|---|---|---|---|---|
| TEMPS DE CULTURE (JOURS) | SUBSTANCE Ic (µg/ml) | | | |
| | 0 | 1 | 10 | 100 |
| 0 - 4 | 12,4 ± 0,96 | 15,9 ± 1,15* | 19,1 ± 1,2* | 21,1 ± 1,0* |
| 4 - 8 | 3,0 ± 0,15 | 3,7 ± 0,15* | 4,4 ± 0,52* | 4,9 ± 0,45* |
| 8 - 12 | 1,04 ± 0,07 | 1,32 ± 0,7* | 1,53 ± 0,15* | 1,75 ± 0,05* |

* : p < 0,025 (U - test de Mann-Whitney)

TABLEAU 4

| Substances n° | Concentrations étudiées (µg/ml) | | | | | | | |
|---|---|---|---|---|---|---|---|---|
| | | 0 | 1 | | 10 | | 100 | |
| | Protéoglycanes libérés durant les 12 jours de culture | | | | | | | |
| | µg /µg ADN /12 jours | | µg /µg ADN /12 jours | | µg /µg ADN /12 jours | | µg /µg ADN /12 jours | |
| | Abs | % | Abs | % | Abs | % | Abs | % |
| Ia | 16 | 100 | 16,4 | 103 | 26,4 | 165 | 33,3 | 208 |
| Ib | 17,6 | 100 | 17,2 | 98 | 26,3 | 149 | 35,2 | 200 |
| Ic | 16,4 | 100 | 20,9 | 127 | 25 | 150 | 27,8 | 170 |

TABLEAU 5

| PG DANS LES AGREGATS de 12 jours (ng/µg ADN) | | | | |
|---|---|---|---|---|
| AGE DE LA CULTURE (JOURS) | SUBSTANCE Ia (µg/ml) | | | |
| | 0 | 1 | 10 | 100 |
| 0 - 12 | 581 ± 98 | 912 ±83,6* | 1411 ± 153* | 2105 ± 42* |

* : p < 0,025 comparé aux témoins
(U - test de Mann-Whitney)

TABLEAU 6

| PG DANS LES AGREGATS de 12 jours (ng/µg ADN) | | | | |
|---|---|---|---|---|
| AGE DE LA CULTURE (JOURS) | SUBSTANCE Ib (µg/ml) | | | |
| | 0 | 1 | 10 | 100 |
| 0 - 12 | 724 ± 222 | 742 ± 62 | 931 ± 24* | 1108 ± 45* |

* : p < 0,025 (U - test de Mann-Whitney)

TABLEAU 7

| PG DANS LES AGREGATS de 12 jours (ng/µg ADN) | | | | |
|---|---|---|---|---|
| AGE DE LA CULTURE (JOURS) | SUBSTANCE Ic (µg/ml) | | | |
| | 0 | 1 | 10 | 100 |
| 0 - 12 | 742 ± 50 | 886 ± 114* | 1046 ± 74* | 1184 ± 33* |

* : p < 0,025 (U - test de Mann-Whitney)

TABLEAU 8

| COLL. II LIBERE DANS LE MILIEU DE CULTURE (ng/µg ADN/96 h) | | | | |
|---|---|---|---|---|
| TEMPS DE CULTURE (JOURS) | SUBSTANCE Ia (µg/ml) | | | |
| | 0 | 1 | 10 | 100 |
| 0 - 4 | 225 ± 22 | 233 ± 25 | 301 ± 52* | 462 ± 51* |
| 4 - 8 | 152 ± 14 | 147 ± 19 | 259 ± 22* | 311 ± 41* |
| 8 - 12 | 97,4 ± 18 | 102,2 ± 10,96 | 147 ± 21* | 109 ± 23* |

* : p < 0,025 (U - test de Mann-Whitney)

TABLEAU 9

| COLL. II LIBERE DANS LE MILIEU DE CULTURE (ng/µg ADN/96 h) | | | | |
|---|---|---|---|---|
| TEMPS DE CULTURE (JOURS) | SUBSTANCE Ib (µg/ml) | | | |
| | 0 | 1 | 10 | 100 |
| 0 - 4 | 204 ± 21 | 222 ± 26 | 322 ± 33* | 392 ± 46* |
| 4 - 8 | 102 ± 12 | 112 ± 19 | 158 ± 14* | 190 ± 22* |
| 8 - 12 | 62,8 ± 6,9 | 59,9 ± 0,6 | 91,7 ± 6,1* | 137,7 ± 13,4* |

* : p < 0,025 (U - test de Mann-Whitney)

TABLEAU 10

| COLL.II LIBERE DANS LE MILIEU DE CULTURE (ng/µg ADN/96 h) | | | | |
|---|---|---|---|---|
| TEMPS DE CULTURE (JOURS) | SUBSTANCE Ic (µg/ml) | | | |
| | 0 | 1 | 10 | 100 |
| 0 - 4 | 107,8 ± 0,3 | 149,4 ± 8,7* | 162 ± 11* | 182 ± 5,6* |
| 4 - 8 | 52,2 ± 5,4 | 65,8 ± 7,4* | 70,5 ± 7,1* | 93,8 ± 10,5* |
| 0 - 12 | 32,2 ± 3,4 | 41,2 ± 4,6* | 50,6 ± 4,2* | 61,1 ± 5,2* |

* : p < 0,025 (U - test de Mann-Whitney)

TABLEAU 11

| Substances n° | Doses étudiées (µg/ml) | | | | | | | |
|---|---|---|---|---|---|---|---|---|
| | 0 | | 1 | | 10 | | 100 | |
| | Libération cumulée du coll.II en 12 jours | | | | | | | |
| | µg /µg ADN /12 jours | | µg /µg ADN /12 jours | | µg /µg ADN /12 jours | | µg /µg ADN /12 jours | |
| | Abs | % | Abs | % | Abs | % | Abs | % |
| Ia | 474 | 100 | 482 | 102 | 787 | 166 | 962 | 202 |
| Ib | 369 | 100 | 394 | 107 | 572 | 155 | 728 | 197 |
| Ic | 191 | 100 | 256 | 134 | 292 | 153 | 337 | 176 |
| Valeur absolue: exprimée en ng/µg ADN/12 jours | | | | | | | | |

TABLEAU 12

| Concentrations moyennes (m ± DS) de collagène de type II dans les agrégats de chondrocytes au terme de 12 jours de culture Doses étudiées | | | | |
|---|---|---|---|---|
| Subs. n° | 0 | 1µg/ml | 10µg/ml | 100µg/ml |
| Ia | 104 ± 15,1 | 105 ± 13 | 165 ± 21** | 175 ± 19** |
| Ib | 90 ± 10 | 98 ± 9 | 121 ± 10** | 158 ± 16** |
| Ic | 61 ± 7 | 81 ± 6* | 94 ± 7** | 121 ± 13** |

* : p < 0.05
** : p <0.01

TABLEAU 13

| Taux des PGE2 (pg/µg d'ADN) dans les milieux de culture prélevés du 4° au 8° jour Doses étudiées | | | | |
|---|---|---|---|---|
| Subs. n° | 0 | 1µg/ml | 10µg/ml | 100µg/ml |
| Ia | 122 ± 39 | 91,5 ± 9 | 44 ± 0,6* | 25 ± 13** |
| Ib | 146 ± 23 | 129 ± 19 | 76 ± 14** | 48 ± 15** |
| Ic | 138 ± 19 | 101 ± 7* | 81 ± 12** | 39 ± 10** |

* : p < 0.025
** : p <0.01

TABLEAU 14

| PG DANS LE MILIEU DE CULTURE (µg/µg ADN - 96 H) | | | | | | | |
|---|---|---|---|---|---|---|---|
| TEMPS DE CULTURE (JOURS) | 0 | Substance Ia (µg/ml) | | Substance Ib (µg/ml) | | Substance Ic (µg/ml) | |
| | | 10 | 100 | 10 | 100 | 10 | 100 |
| 0 - 4 | 6,5 ± 0,75 (100%) | 7,7 ± 0,49* (118,5%) | 9,5 ± 1,34* (147%) | 7,6 ± 0,41* (117%) | 11,9 ± 2,09* (183,5%) | 7,8 ± 1,51* (120%) | 10,4 ± 1,19* (160%) |
| 4 - 8 | 1,99 ± 0,19 (100%) | 2,37 ± 0,17* (119%) | 2,69 ± 0,15* (135%) | 2,32 ± 0,21* (117%) | 2,71 ± 0,24* (136%) | 2,34 ± 0,08* (118%) | 2,40 ± 0,35* (121%) |

* : p < 0,025 comparé aux témoins non traités (U -test de Mann-Whitney)

TABLEAU 15

| PG DANS LE MILIEU DE CULTURE (µg/µg ADN - 96 H) SUBSTANCE (µg/ml) | | | | | | | |
|---|---|---|---|---|---|---|---|
| TEMPS DE CULTURE (JOURS) | 0 | Ia | | Ib | | Ic | |
| | | 10 | 100 | 10 | 100 | 10 | 100 |
| 0 - 4 | 5,3 ± 0,38 (100%) | 6,9 ± 0,99* (131%) | 8,8 ± 1,49* (166%) | 7,1 ± 0,36* (134%) | 7,9 ± 0,30* (149%) | 7,0 ± 1,19* (132%) | 7,5 ± 0,96* (140%) |
| 4 - 8 | 1,7 ± 0,19 (100%) | 2,1 ± 0,21* (123%) | 2,4 ± 0,09* (138%) | 2,1 ± 0,16* (125%) | 2,3 ± 0,13* (137%) | 2,0 ± 0,17* (117) | 2,4 ± 0,25* (138%) |

* : p < 0,025 (U - test de Mann-Whitney)

TABLEAU 16

| PG DANS LES AGREGATS de 8 jours (ng/µg DNA) | | | | | | | |
|---|---|---|---|---|---|---|---|
| TEMPS DE CULTURE (JOURS) | 0 | Substance Ia (µg/ml) | | Substance Ib (µg/ml) | | Substance Ic (µg/ml) | |
| | | 10 | 100 | 10 | 100 | 10 | 100 |
| 0 - 8 | 784 ± 105 (100%) | 1004 ± 56* (128%) | 1242 ± 208* (158%) | 1108 ± 166* (141%) | 1142 ± 110* (146%) | 915 ± 75* (117%) | 1035 ± 121* (132%) |

* : $p < 0{,}025$ comparé aux témoins non traités
(U - test de Mann-Whitney)

TABLEAU 17

| PG DANS LES AGREGATS (ng/µg ADN) de 8 jours | | | | | | | |
|---|---|---|---|---|---|---|---|
| TEMPS DE CULTURE (JOURS) | 0 | Substance Ia (µg/ml) | | Substance Ib (µg/ml) | | Substance Ic (µg/ml) | |
| | | 10 | 100 | 10 | 100 | 10 | 100 |
| 0 - 8 | 576,0± 40,55 (100%) | 688,8± 40,65* (120%) | 829,4± 9,37* (144%) | 724,0± 72,45* (126%) | 832,0± 59,10* (144%) | 718,2± 62,61* (125%) | 799,5± 85,9* (139%) |

* : $p < 0{,}025$ comparé aux témoins non traités

TABLEAU 18

| COLL. II LIBERE DANS LE MILIEU DE CULTURE (ng/µg ADN/96 h) | | | | | | | |
|---|---|---|---|---|---|---|---|
| TEMPS DE CULTURE (JOURS) | 0 | Substance Ia (µg/ml) | | Substance Ib (µg/ml) | | Substance Ic (µg/ml) | |
| | | 10 | 100 | 10 | 100 | 10 | 100 |
| 0 - 4 | 32,7 ± 5,59 | 44,3 ± 2,30* | 77,0± 15,58* | 41,4 ± 0,51* | 81,1 ± 20,2* | 44,1 ± 6,98* | 70,7 ± 10,3* |
| 4 - 8 | 25,9 ± 5,10 | 34,0 ± 3,11* | 47,2 ± 5,19* | 33,3 ± 4,61* | 55,0± 17,21* | 33,5 ± 3,71* | 41,7 ± 3,69* |

* : $p < 0{,}025$ comparé aux témoins non traités
(U - test de Mann-Whitney)

TABLEAU 19

| COLL. II DANS LE MILIEU DE CULTURE (ng/µg ADN/96 h) | | | | | | | |
|---|---|---|---|---|---|---|---|
| TEMPS DE CULTURE (JOURS) | 0 | Substance Ia (µg/ml) | | Substance Ib (µg/ml) | | Substance Ic (µg/ml) | |
| | | 10 | 100 | 10 | 100 | 10 | 100 |
| 0 - 4 | 23,0 ± 3,91 | 37,4 ± 1,98* | 44,2 ± 11,6* | 38,2 ± 2,60* | 56,3 ± 12,41* | 37,9 ± 5,47* | 53,2 ± 4,21* |
| 4 - 8 | 16,3 ± 1,72 | 25,3 ± 2,20* | 38,2 ± 5,71* | 29,6 ± 3,44* | 46,3 ± 2,61* | 23,3 ± 2,11* | 33,5 ± 1,73* |

* : p < 0,025 (U - test de Mann-Whitney)

TABLEAU 20

| COLL. II DANS LES AGREGATS (ng/µg ADN) | | | | | | | |
|---|---|---|---|---|---|---|---|
| TEMPS DE CULTURE (JOURS) | 0 | Substance Ia (µg/ml) | | Substance Ib (µg/ml) | | Substance Ic (µg/ml) | |
| | | 10 | 100 | 10 | 100 | 10 | 100 |
| 4 - 8 | 62,8 ± 4,90 | 78,8 ± 10,5* | 114,7 ± 10,55* | 81,6 ± 16,00* | 113,2 ± 18,25* | 85,9 ± 14,45* | 109,8 ± 8,52* |

* : p < 0,025 (U - test de Mann-Whitney)

TABLEAU 21

| $PGE_2$ DANS LE MILIEU DE CULTURE (pg/µg ADN/96 h) | | | | | | | |
|---|---|---|---|---|---|---|---|
| TEMPS DE CULTURE (JOURS) | 0 | Substance Ia (µg/ml) | | Substance Ib (µg/ml) | | Substance Ic (µg/ml) | |
| | | 10 | 100 | 10 | 100 | 10 | 100 |
| 0 - 4 | 300 ± 17 | 312 ± 15 | 143 ± 25* | 130 ± 21* | 121 ± 16* | 160 ± 17* | 153 ± 15* |
| 4 - 8 | 48 ± 3,5 | 27 ± 2,5* | 25 ± 3,5* | 30 ± 1,7* | 25 ± 1* | 35,5 ± 2,1* | 29 ± 3,5* |

* : p < 0,025 comparé aux témoins non traités
(U - test de Mann-Whitney)

TABLEAU 22

| Inhibition (exprimée en % par rapport aux témoins) de la production des radicaux Hydroxyle par les substances Ia, Ib et Ic. Substrat: KMB | | | |
|---|---|---|---|
| | % D'INHIBITION | | |
| Produits Concentration | Ia | Ib | Ic |
| $10^{-3}$ M | 78 | 80 | 74 |
| $10^{-4}$ M | 41 | 46 | 39 |
| $10^{-5}$ M | 22 | 25 | 20 |

TABLEAU 23

| Inhibition (exprimée en % par rapport aux témoins) de la production des radicaux Hydroxyle par les substances Ia, Ib et Ic. Substrat: Acide linoléique | | | |
|---|---|---|---|
| | % D'INHIBITION | | |
| Produits Concentration | Ia | Ib | Ic |
| $10^{-3}$ M | 94 | 93 | 91 |
| $10^{-4}$ M | 42 | 45 | 40 |
| $10^{-5}$ M | 0 | 0 | 0 |

**REFERENCES**


Bassleer C., Gysen P., Foidart J.M., Bassleer R., Franchimont P.: Human chondrocytes in tridimensional culture. In vitro, 1986, **22**: 113-119

Bayliss M.T., Venn M.: Chemistry of human articular cartilage. In: Maroudas A., Holborow E.J., eds. Studies in joint diseases, 1st ed. London: Pitman Medical, 1980, 2-58

Franchimont P., Bouffioux Ch., Reuter A. et al. Radioimmunoassay of prostatic acid phosphatase: validation and clinical application. Int. J. Cancer. 1983, 89: 114-124

Franchimont P., Bassleer C., Henrotin Y., Gysen P., Bassleer R.: Effects of human and salmon calcitonin in human articular chondrocytes cultivated in clusters. J. Clin. Endocr. Metab., 1989, 69: 259-266

Franchimont P., Bassleer C.: New diagnostic tools and methodological approaches; an outlook to the future. Scand. J. of Rheumatol., 1989, 80: 29-31.

Franchimont P., Bassleer C.: Effects of hormones and local growth factors on articular chondrocyte metabolism. Scand. J. of Rheumatol., 1991, suppl.27, 18: 68: 70

Greenwood F.C., Hunter W., Glover J. : The preparation of $^{131}$I-labelled human growth hormone of high specific radioactivity. Biochem J. 1973, 89: 114-24

Gysen P., Franchimont P. : Radioimmunoassay of proteoglycans J. Immunoassay., 1984, 5: 221-243

Henrotin Y., Bassleer C., Collette J., Nusgens B., Franchimont P.: Radioimunoassay for human type II collagen. J. of Immunoassay, 1990, 11(4): 555-578

Herbage D., Bouillet J., Bernengo J.C. : Biochemical and physicochemical characterization of pepsin-solubilized type II collagen from bovine articular cartilage. Biochem. J. 1977, 161: 303-312

Labarca C., Paigen K.: A simple, rapide and sensitive DNA assay procedure. Anal. Biochem., 1980, 102: 344-352

Oegema T.R., Hascall V.C., Dziewatkowski D.D.: Isolation and characterization of proteoglycans from the swarm rat chondrosarcoma. J. Biol. Chem. 1975, 250: 6151-6159

Roughley P.J., Mc Nicol D., Santer V., Buckwalter J. The presence of a cartilage-like proteoglycan in the adult human meniscus. Biochem. J. 1981. 197: 77-83

Salacynsky P., Hope J., Mc Lean C. et al.: A new simple method which allows theoretical incorporation of radio-iodine into proteins and peptides without damage. J. Endocrinol. 1979, 81: 131

Serteyn D., Deby-Dupont G., Pincemail J., Mottart E., Philippart C., Lamy M.: Equine post-anaesthesic myositis: thromboxane, prostacyclin and prostaglandin E2 production. Vet. Res. Com., 1988, 12: 219-226

Vaitukaitis J., Robbins J.B., Nieschlag E., Ross G.T.: A method for producing specific antisera with small doses of immunogen. J Clin. Endocrinol. Metab. 1971, 33: 988-91

Weiss S.S., Rustagi P.K., Lobuglio A.F.: Human generation of hydroxyl radical. J. Exp. Med., 1978, 147: 316-324

## Revendications

1. Utilisation de prodelphinidines de formule (I)

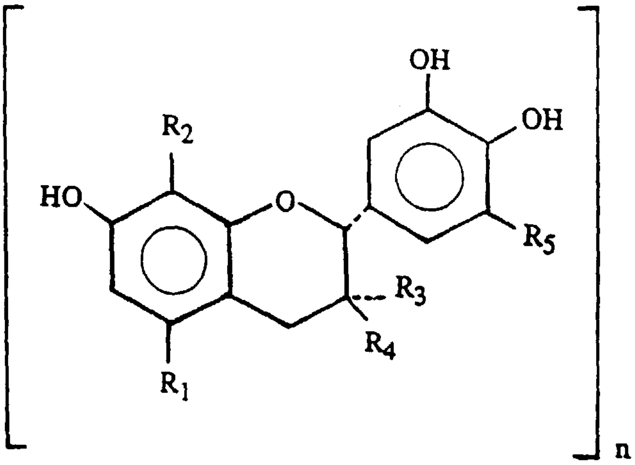

(I)

dans laquelle:

- $R_1$ et $R_2$ représentent indépendamment H ou OH,
- $R_3$ et $R_4$ sont différents et représentent H ou OH,
- $R_5$ représente H (catéchine/épicatéchine) ou OH (gallocatéchine/épigallocatéchine), et,
- n est un nombre entier de 2 à 40,
- sous réserve que l'une au moins des 2 à 40 unités décrites ci-dessus, correspond à une gallocatéchine ($R_3$ = H, $R_4$ = OH), ou à une épigallocatéchine ($R_3$ = OH, $R_4$ = R), pour laquelle $R_5$ représente OH, pour l'obtention de médicaments destinés au traitement des pathologies articulaires liées à une destruction du cartilage, et plus particulièrement de l'arthrose.

2. Utilisation selon la revendication 1, caractérisée en ce que les prodelphinidines sont choisies parmi celles répondant à la formule (II) suivante:

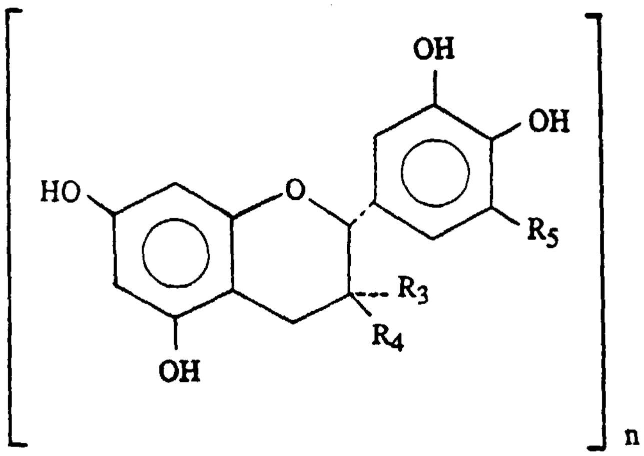

(II)

dans laquelle $R_3$, $R_4$ et $R_5$ sont tels que définis dans la revendication 1.

**3.** Utilisation selon la revendication 1 ou la revendication 2, caractérisée en ce que les médicaments sont destinés à l'administration par voie orale. parentérale ou topique.

**4.** Utilisation selon l'une des revendications 1 à 3, caractérisée en ce que les prodelphinidines sont celles issues de Ribes species, et plus particulièrement de Ribes nigrum.

**5.** Utilisation selon la revendication 4, caractérisée en ce que les prodelphinidines sont contenues dans des extraits de Ribes species, et plus particulièrement de Ribes nigrum, obtenus à partir des méthodes classiques en champs ou bien par des techniques de production in vitro.

**6.** Utilisation selon la revendication 5, caractérisée en ce que les prodelphinidines sont isolées et purifiées à partir d'extraits de Ribes species, et plus particulièrement de Ribes nigrum, obtenus à partir des méthodes classiques en champs ou bien par des techniques de production in vitro.

**7.** Utilisation selon l'une des revendications 1 à 6, caractérisée en ce que les prodelphinidines renferment l'une au moins des molécules (Ia), (Ib), et (Ic) suivantes:

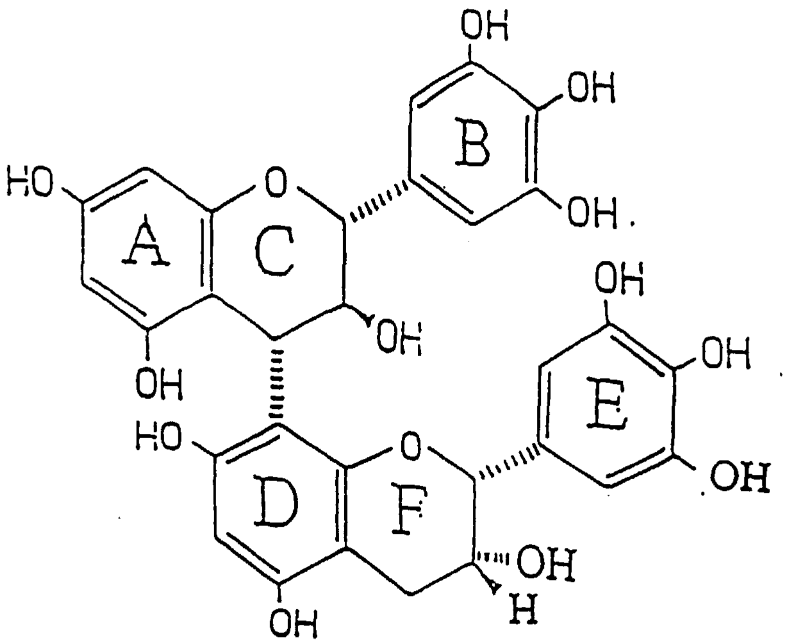

(Ia)

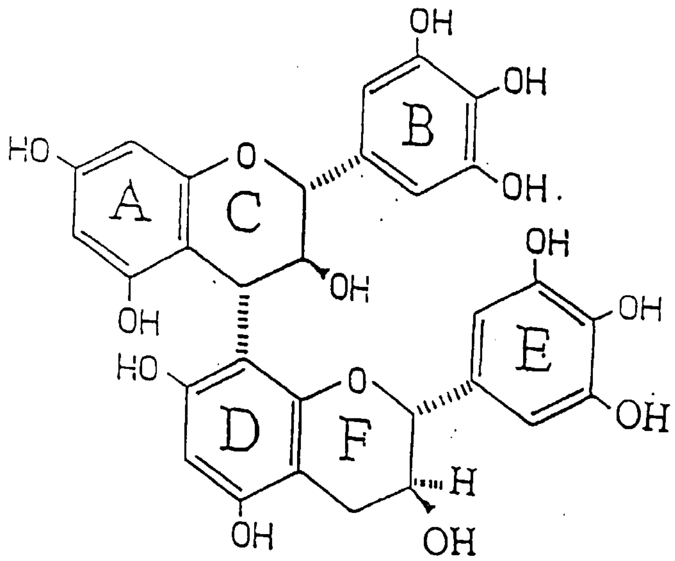

(Ib)

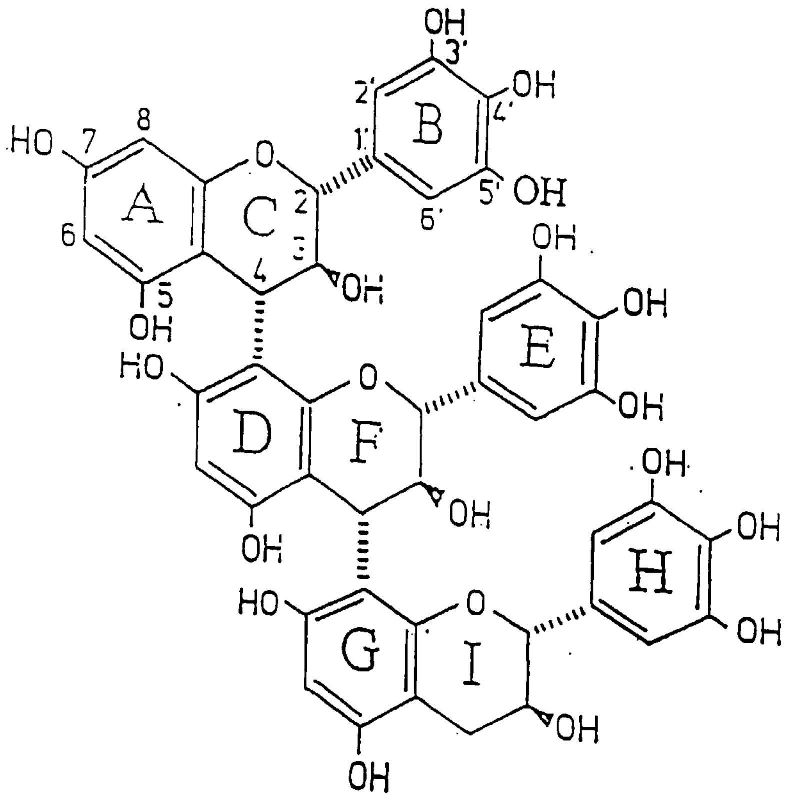

(Ic)

## Claims

**1.** Use of prodelphinidins of formula (I):

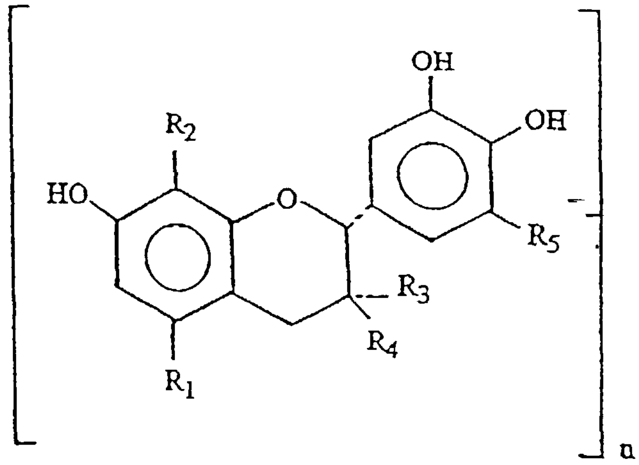

(I)

in which:

- $R_1$ and $R_2$ independently represent H or OH,
- $R_3$ and $R_4$ are different and represent H or OH,
- $R_5$ represents H (catechin/epicatechin) or OH (gallocatechin/epigallocatechin), and
- n is an integer from 2 to 40,
- under the condition that at least one of the 2 to 40 units described above corresponds to a gallocatechin ($R_3$=H, $R_4$=OH), or to an epigallocatechin ($R_3$=OH, $R_4$=H), for which $R_5$ represents OH,
  for obtaining medicaments intended for the treatment of articular pathologies associated with destruction of the cartilage, and more particularly of arthrosis.

**2.** Use according to Claim 1, characterized in that the prodelphinidins are chosen from those corresponding to the following formula (II):

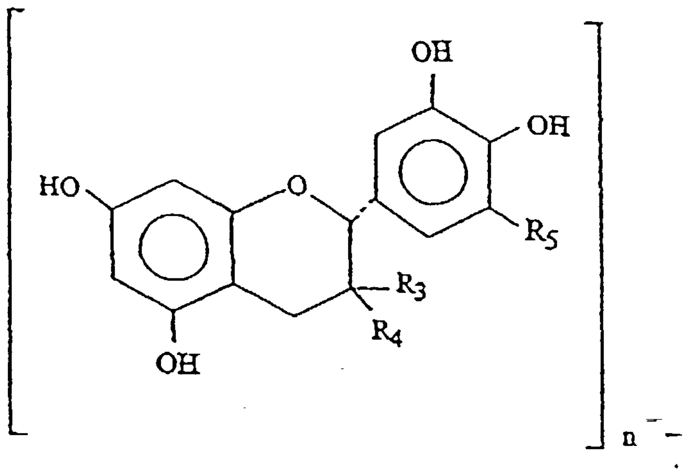

(II)

in which $R_3$, $R_4$ and $R_5$ are as defined in Claim 1.

**3.** Use according to Claim 1 or Claim 2, characterized in that the medicaments are intended for administration by the oral, parenteral or topical route.

**4.** Use according to one of Claims 1 to 3, characterized in that the prodelphinidins are those orginating from the Ribes species, and more particularly from Ribes nigrum.

**5.** Use according to Claim 4, characterized in that the prodelphinidins are contained in extracts of the Ribes species, and more particularly from Ribes nigrum, obtained by methods conventional in the field or by in vitro production techniques.

**6.** Use according to Claim 5, characterized in that the prodelphinidins are isolated and purified from extracts of the Ribes species, and more particularly from Ribes nigrum, obtained by methods conventional in the field or by in vitro production techniques.

**7.** Use according to one of Claims 1 to 6, characterized in that the prodelphinidins contain at least one of the following molecules (Ia), (Ib) and (Ic):

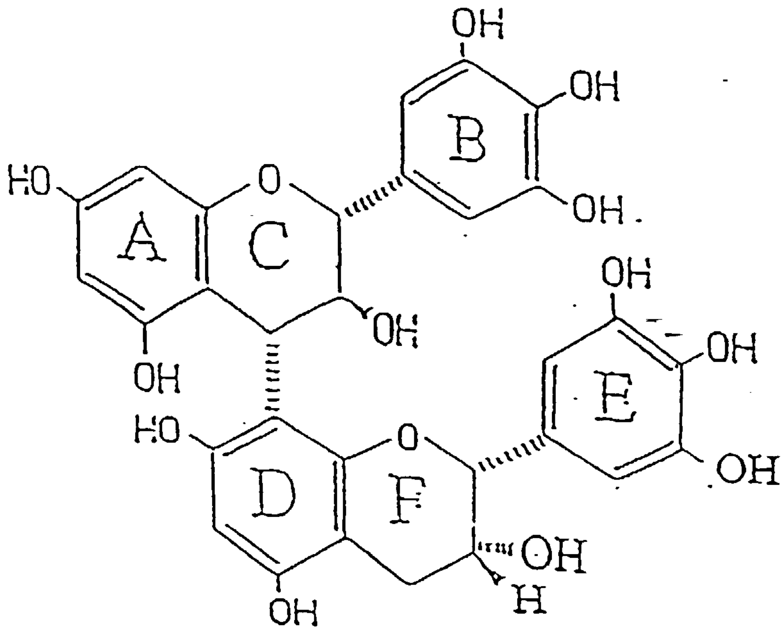

(Ia)

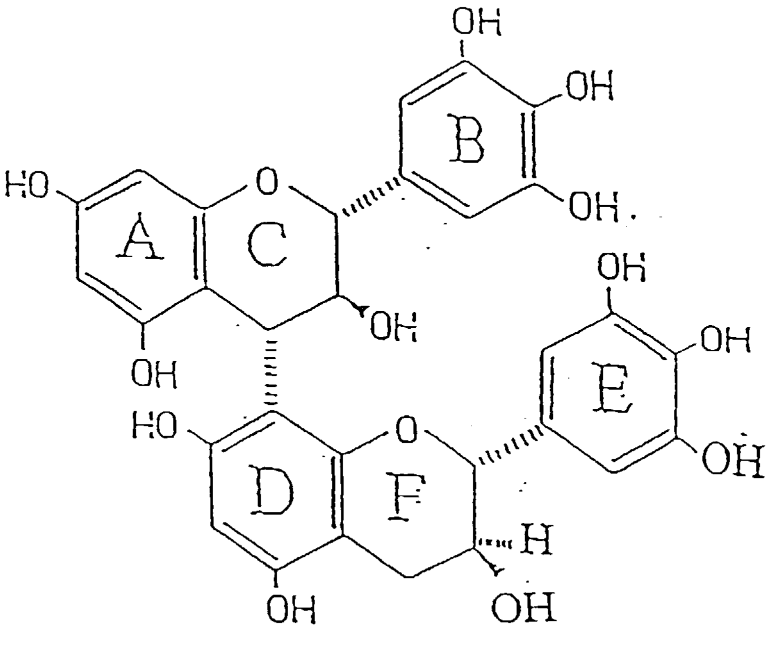

(Ib)

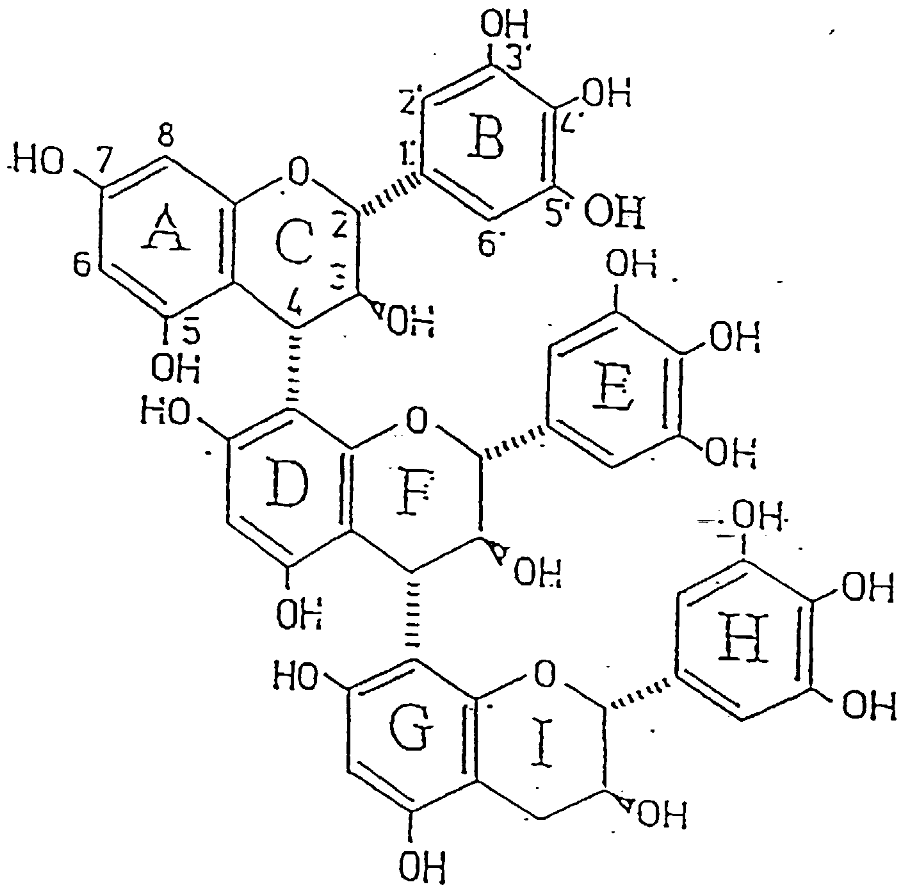

(Ic)

**Patentansprüche**

1. Verwendung von Prodelphinidinen der Formel (I)

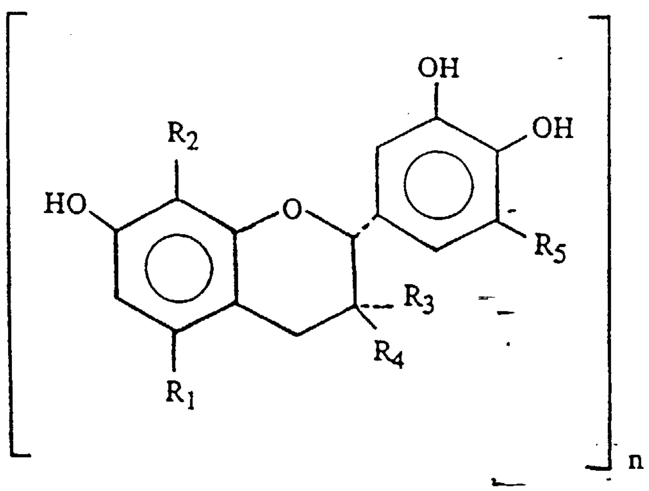

(I)

wobei:

- $R_1$ und $R_2$ unabhängig voneinander H oder OH darstellen,
- $R_3$ und $R_4$ verschieden sind und H oder OH darstellen,
- $R_5$ H (Katechin/Epikatechin) oder OH (Gallokatechin/Epigallokatechin) darstellt, und
- n eine ganze Zahl von 2 bis 40 ist,
- unter der Voraussetzung, daß mindestens eine der oben beschriebenen 2 bis 40 Einheiten einem Gallokatechin ($R_3$ = H, $R_4$ = OH) oder einem Epigallokatechin ($R_3$ = OH, $R_4$ = H) entspricht, bei dem $R_5$ OH darstellt, zur Herstellung von Medikamenten, die zur Behandlung von Gelenkerkrankungen bestimmt sind, die mit einer Zerstörung des Knorpels zusammenhängen, und genauer gesagt zur Behandlung von Arthrose.

**2.** Verwendung nach Anspruch 1, dadurch charakterisiert, daß die Prodelphinidine ausgewählt werden unter denen, die der folgenden Formel (II) entsprechen:

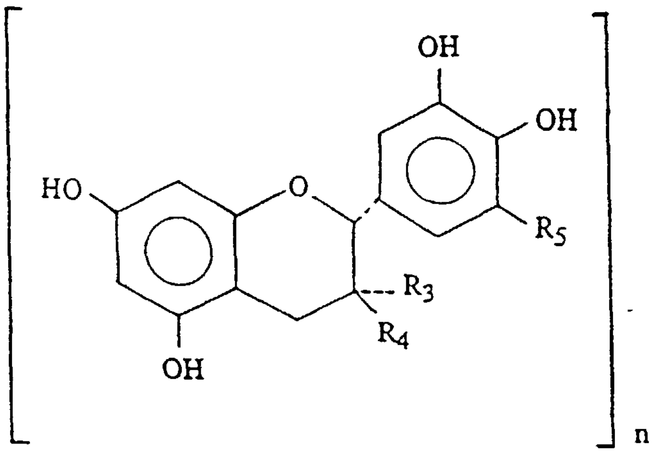

wobei $R_3$, $R_4$ und $R_5$ wie in Anspruch 1 definiert sind.

**3.** Verwendung nach Anspruch 1 oder nach Anspruch 2, dadurch charakterisiert, daß die Medikamente zur Verabreichung auf oralem, parenteralem oder topischem Weg bestimmt sind.

**4.** Verwendung nach einem der Ansprüche 1 bis 3, dadurch charakterisiert, daß die Prodelphinidine diejenigen sind, die aus Ribes-Spezies stammen, und genauer gesagt aus Ribes nigrum.

**5.** Verwendung nach Anspruch 4, dadurch charakterisiert, daß die Prodelphinidine in Extrakten von Ribes-Spezies enthalten sind, und genauer gesagt von Ribes nigrum, die erhalten werden durch klassische Feldmethoden oder durch in vitro-Herstellungstechniken.

**6.** Verwendung nach Anspruch 5, dadurch charakterisiert, daß die Prodelphinidine ausgehend von Extrakten von Ribes-Spezies, und genauer gesagt von Ribes nigrum isoliert und gereinigt werden, die mit klassischen Feldmethoden oder durch in vitro-Herstellungsverfahren erhalten werden.

**7.** Verwendung nach einem der Ansprüche 1 bis 6, dadurch charakterisiert, daß die Prodelphinidine mindestens eines der folgenden Moleküle (Ia), (Ib) und (Ic) beinhalten:

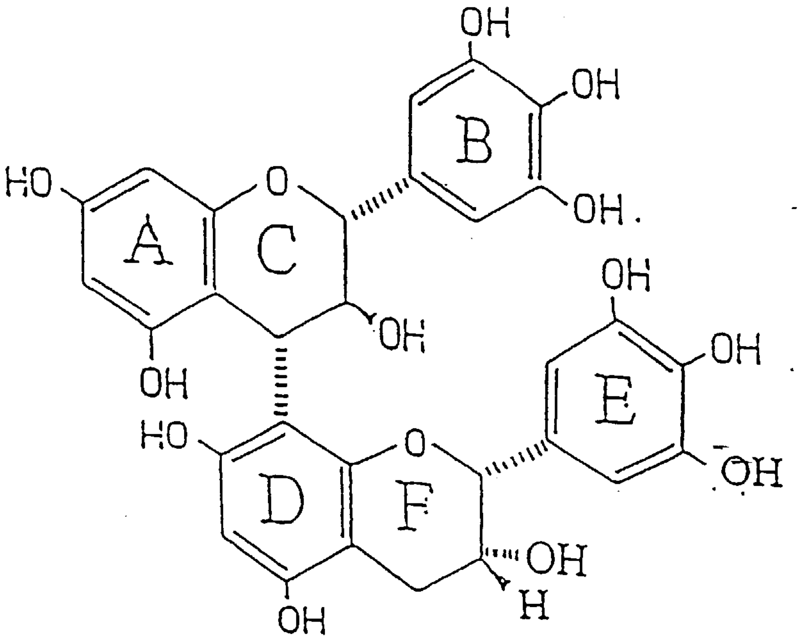

(Ia)

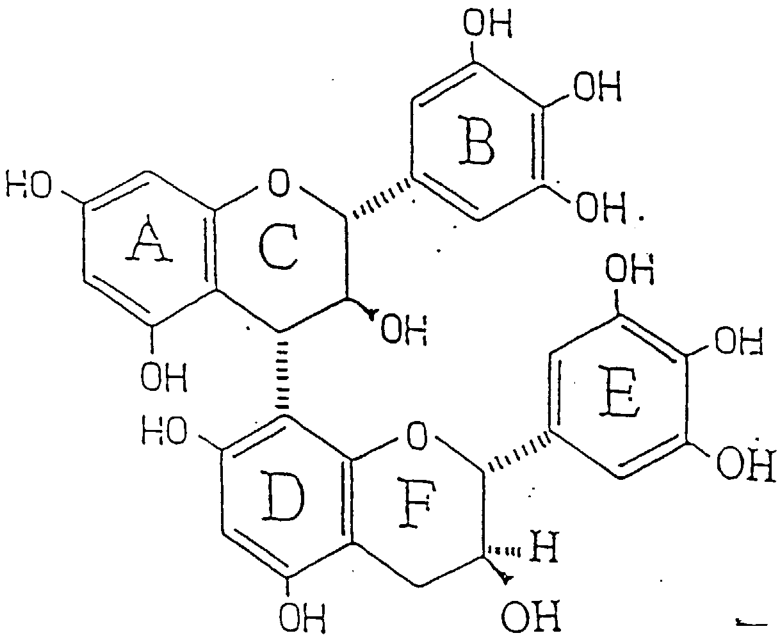

(Ib)

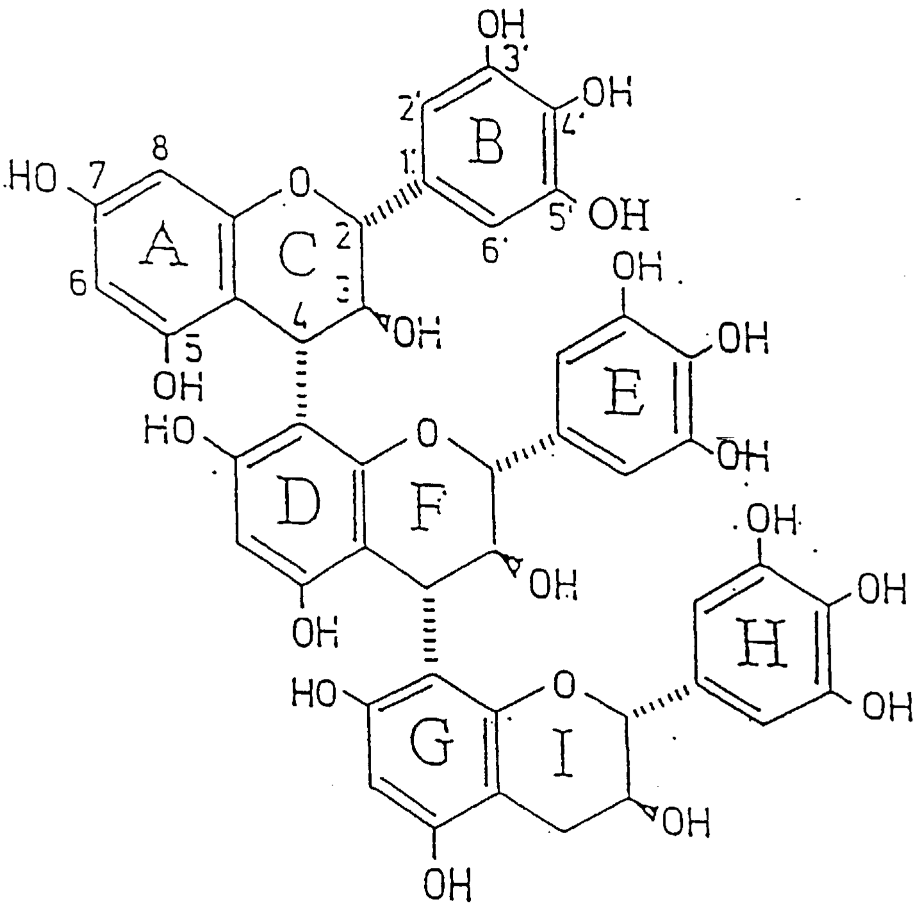

(Ic)